# EUROPEAN PATENT APPLICATION

(11) **EP 4 570 183 A1**
(43) Date of publication of application: **18.06.2025**
(21) Application number: 23307179.4
(22) Date of filing: 12.12.2023
(51) Int. Cl.: A61B 8/06, A61B 8/08

(54) **DETERMINING COLOR MAPPING FUNCTIONS**

(71) Applicant: Koninklijke Philips N.V., 5656 AG Eindhoven (NL)
(72) Inventor: SCHRECKENBERG, Marcus, 5656 Eindhoven (NL); BONNEFOUS, Odile, 5656 Eindhoven (NL); ROEDIGER, Verena, 5656 Eindhoven (NL); ROUET, Jean-Michel, 5656 Eindhoven (NL); BRAUN, Isabella, 5656 Eindhoven (NL); BLANKENHAGEN, Michael, 5656 Eindhoven (NL); DECOMBAS-DESCHAMPS, Sofiane, 5656 Eindhoven (NL)
(74) Representative: Philips Intellectual Property & Standards

(57) **Abstract**

Proposed concepts thus aim to provide schemes, solutions, concept, designs, methods and systems pertaining to dynamically determining a color mapping function for Doppler data of regurgitant flow in a cardiac valve. In particular, embodiments aim to provide a method for dynamically determining a color mapping function for Doppler data of regurgitant flow in a cardiac valve by obtaining and processing Doppler data comprising at least two Doppler data frames representing regurgitant flow associated with an orifice in a cardiac valve, and a blood flow model of the regurgitant flow. In other words, it is proposed that, for each Doppler data frame of the Doppler data, by determining a region of interest in the blood flow model based on the blood flow model and the color map, a target iso-velocity surface of the model can be determined, and a color mapping function for said Doppler data frame can be determined based on this target iso-velocity surface.

## Description

### FIELD OF THE INVENTION

This invention relates to the field of color mapping functions, and more specifically, to the field of color mapping functions for Doppler data.

### BACKGROUND OF THE INVENTION

In mitral regurgitation (MR), the mitral valve does not close properly and therefore allows backflow of blood from the left ventricle into the left atrium when the left ventricle contracts (systole). Based on Doppler velocities, an algorithm can create a model of the fluid dynamics. Based on this model, the flow for each frame and the overall regurgitant flow volume can be calculated.

Quantifying mitral regurgitation orifice area and flow volume is clinically important, but ultrasound manual techniques are difficult and often inaccurate. Some algorithms have been developed for automatic estimation of orifice area and flow volume which iteratively adjust a model of orifice geometry and proximal flow convergence of a proximal iso-velocity surface area (PISA) method to match 3D color flow data.

However, the results from common methods of analyzing mitral regurgitation flow (or any other cardiac valve regurgitation) often involve crude simplifications and thus lead to inaccurate and/or unreliable results. Furthermore, it is often difficult or impossible for a user to review and assess the correctness of the algorithmic results for every frame, in particular, due to inadequate color mapping functions for visualizing the Doppler data. Due to this, it is not currently possible for a user to assess the output of algorithms to determine whether they have processed the data correctly. Furthermore, volumetric data is even more difficult for a user to review as inner values (or colors in the case of 3D color maps) can be obscured by outer values.

### SUMMARY OF THE INVENTION

The invention is defined by the claims.

According to examples in accordance with an aspect of the invention, there is provided a method for dynamically determining a color mapping function for Doppler data of regurgitant flow in a cardiac valve.

The method comprises: obtaining Doppler data, wherein the Doppler data comprises at least two Doppler data frames from a temporal sequence and is representative of regurgitant flow associated with an orifice in a cardiac valve, and wherein each Doppler data frame comprises velocity values; obtaining a blood flow model of the regurgitant flow, wherein the blood flow model comprises a model of the orifice and blood flow velocity values through and around the orifice; and for each Doppler data frame of the Doppler data: determining a region of interest in the blood flow model based on velocity values of the blood flow model and the Doppler data frame; determining a target iso-velocity surface of the blood flow model based on data of the blood flow model within the region of interest; and determining a color mapping function for the Doppler data frame based on the target iso-velocity surface, wherein the color mapping function is for mapping velocity values of the Doppler data frame to colors.

Proposed concepts thus aim to provide schemes, solutions, concept, designs, methods and systems pertaining to dynamically determining a color mapping function for Doppler data of regurgitant flow in a cardiac valve. In particular, embodiments aim to provide a method for dynamically determining a color mapping function for Doppler data of regurgitant flow in a cardiac valve by obtaining and processing Doppler data comprising at least two Doppler data frames representing regurgitant flow associated with an orifice in a cardiac valve, and a blood flow model of the regurgitant flow.

In other words, it is proposed that, for each Doppler data frame of the Doppler data, by determining a region of interest in the blood flow model based on the blood flow model and the Doppler data frame, a target iso-velocity surface of the model can be determined. A color mapping function for said Doppler data frame can then be determined based on this target iso-velocity surface.

By following this process for each Doppler data frame of the Doppler data, color mapping functions for the Doppler data can essentially be dynamically and automatically determined such that, in contrast to methods in the prior art, color renderings of each Doppler data frame can be generated, each with a potentially different color mapping function and without requiring any user intervention. This facilitates the optimization of determining color mapping functions for each Doppler data frame in a temporal sequence of Doppler data rather than trying to optimize a single color mapping function for all the Doppler data frames or requiring user input to manually adjust color mapping functions for each frame. A user can thus be automatically provided with information contained within the Doppler data and the blood flow model that they otherwise would not have had access to, e.g., would not have been able to interpret.

For example, a color mapping function can be determined for each Doppler data frame of the Doppler data such that the regurgitant flow in each subsequently generated color rendering (of each Doppler data frame using their corresponding determined color mapping function) can be made present / clearer. In other words, determining a color mapping function for each Doppler data frame can facilitate dynamic aliasing baselines across subsequently generated color renderings/maps of each Doppler data frame such that the regurgitant flow can be made visible (or more visible) to a user across the entirety of the temporal sequence of Doppler data frames. In contrast, current methods typically analyze a temporal sequence of Doppler data in its entirety and produce a single color mapping function which is then used to generate a color rendering for each frame. This, however, leads to sub-optimal provision of information in each color rendering such that important information may be lost in some color renderings as the aliasing baseline and/or color range of the color renderings, for example, is not suitable for said Doppler data frame.

The inventors have realized that by using both Doppler data and a blood flow model, an iso-velocity surface of the blood flow model for the Doppler velocity values associated with each Doppler data frame can be found that allows a beneficial determination of a color mapping function for each Doppler data frame. In this way, a user can be provided with beneficial information which they otherwise would not have had access to, e.g., the color mapping function can be used to reveal information in, for example, a subsequent color map that can then facilitate a user being able to compare and/or match the Doppler data to the blood flow model.

Ultimately, an improved method for dynamically adjusting Doppler data frames of regurgitant flow in a cardiac valve is provided.

In some embodiments, the at least two Doppler data frames from the temporal sequence may be temporally adjacent. This allows for two adjacent frames from Doppler data, for example, to each have their own determined color mapping function such that, for example, subsequently generated color renderings (such as color maps) for the Doppler data are essentially being dynamically adjusted on a frame-by-frame basis. This essentially facilitates, for example, each frame of the Doppler data/video having a more optimal color rendering.

In some embodiments, the method may further comprise generating a color rendering for a Doppler data frame based on the velocity values of said Doppler data frame and the determined color mapping function for said Doppler data frame. In this way, a user may be provided with a color rendering generated using a more beneficial/optimal color mapping function.

In some embodiments, the method may further comprise generating a display control signal, the display control signal describing the color rendering for the Doppler data frame. In this way, a user may be presented with a generated color rendering.

In some embodiments, the display control signal may further describe the color mapping function of said Doppler data frame. In this way, a user can analyze the color rendering in the context of the color mapping function which might be, for instance, a look-up table for a color map, to see, for example, what velocity the aliasing baseline is representing.

In some embodiments, the color mapping function may comprise a look-up table. This is an efficient and/or effective form of color mapping function.

In some embodiments, the color mapping function may be configured to map at least one velocity value to a transparent color. This can facilitate the generation of color renderings which can highlight, for example, a subset of velocity values or even a single velocity value by, for example, making other velocity values appear transparent in the color rendering, i.e., have no color.

In some embodiments, determining a region of interest in the blood flow model may comprise comparing velocity values of the blood flow model to velocity values of the Doppler data frame; and determining a region of interest in the blood flow model based on the comparison. In this way, for example, the region where the different velocities best match, i.e., the region where the voxels of the blood flow model are most reliable, can be used to determine a target iso-velocity surface.

In some embodiments, determining the color mapping function of the Doppler data frame may comprise determining the color mapping function based on the velocity of the target iso-velocity surface. For example, the color mapping function may be determined such that, for example, an aliasing baseline of a color rendering of said Doppler data frame generated using said color mapping function is at the velocity of the target iso-velocity surface.

In some embodiments, the velocity of the target iso-velocity surface may describe the average velocity of the region of interest. This is a beneficial parameter of the iso-velocity surface to use.

In some embodiments, the region of interest may be further based on a requirement that the region of interest is upstream of the cardiac valve. This facilitates determining color mapping functions based on regurgitant flow upstream of the cardiac valve, as opposed to flow downstream of the cardiac valve.

In some embodiments, the region of interest may be further based on a requirement that the region of interest is within a predetermined distance of the cardiac valve. This may ensure that the region of interest is close to the cardiac valve, thus providing more beneficial data for adjusting the color maps.

In some embodiments, prior to determining a region of interest, the method may further comprise adjusting the blood flow model based on the Doppler data. This allows the blood flow model to be adjusted to, for example, better correspond to the actual Doppler data.

In some embodiments, determining a target iso-velocity surface may comprise determining a target iso-velocity surface of the blood flow model based on velocity values of the Doppler data within the region of interest. This facilitates the use of beneficial information for determining the target iso-velocity surface.

In some embodiments, the display control signal may further describe the Doppler data. In this way, a user may be presented with the actual Doppler data as well as the generated color renderings for better context and understanding of both.

In some embodiments, the method may further comprise generating a mesh representation of a proximal flow convergence of the blood flow model; and wherein the display control signal may further describe the mesh representation. In this way, a user may be presented with further useful representations of the data for better context and understanding.

According to another aspect of the invention, there is provided a computer program comprising code means for implementing the method of any herein disclosed method when said program is run on a processing system.

According to another aspect of the invention, there is provided a system for dynamically determining a color mapping function for Doppler data of regurgitant flow in a cardiac valve. The system comprising an input interface configured to: obtain Doppler data, wherein the Doppler data comprises at least two Doppler data frames from a temporal sequence and is representative of regurgitant flow associated with an orifice in a cardiac valve, and wherein a Doppler data frame comprises velocity values; obtain a blood flow model of the regurgitant flow, wherein the blood flow model comprises a model of the orifice and blood flow velocity values through and around the orifice; and a processing unit configured to: for each Doppler data frame of the Doppler data: determine a region of interest in the blood flow model based on velocity values of the blood flow model and the Doppler data frame; determine a target iso-velocity surface of the blood flow model based on data of the blood flow model within the region of interest; and determining a color mapping function for the Doppler data frame based on the target iso-velocity surface, wherein the color mapping function is for mapping velocity values of the Doppler data frame to colors.

Thus, there may be proposed concepts for dynamically determining a color mapping function for Doppler data of regurgitant flow in a cardiac valve, and this may be done based on obtained Doppler data comprising at least two Doppler data frames representing regurgitant flow associated with an orifice in a cardiac valve, and a blood flow model of the regurgitant flow.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for dynamically determining a color mapping function for Doppler data of regurgitant flow in a cardiac valve according to a proposed embodiment;
Fig. 2 is a diagram showing regurgitant flow in a cardiac valve;
Fig. 3 is a flow diagram of a method for dynamically determining a color mapping function for Doppler data of regurgitant flow in a cardiac valve according to a proposed embodiment;
Fig. 4 is a flow diagram of a method for dynamically determining a color mapping function for Doppler data of regurgitant flow in a cardiac valve according to a proposed embodiment;
Fig. 5 is a simplified block diagram of a system for dynamically determining a color mapping function for Doppler data of regurgitant flow in a cardiac valve according to a proposed embodiment; and
Fig. 6 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to dynamically determining a color mapping function for Doppler data of regurgitant flow in a cardiac valve. According to proposed concepts, a number of possible solutions may be implemented separately or jointly. That is, although these possible solutions may be described below separately, two or more of these possible solutions may be implemented in one combination or another.

Embodiments of the invention aim to provide a method for dynamically determining a color mapping function for Doppler data of regurgitant flow in a cardiac valve. This can be achieved by obtaining and processing Doppler data comprising at least two Doppler data frames representing regurgitant flow associated with an orifice in a cardiac valve, and a blood flow model of the regurgitant flow.

In other words, it is proposed that, for each Doppler data frame of the Doppler data, by determining a region of interest in the blood flow model based on the blood flow model and the Doppler data frame, a target iso-velocity surface of the model can be determined. A color mapping function for said Doppler data frame can then be determined based on this target iso-velocity surface.

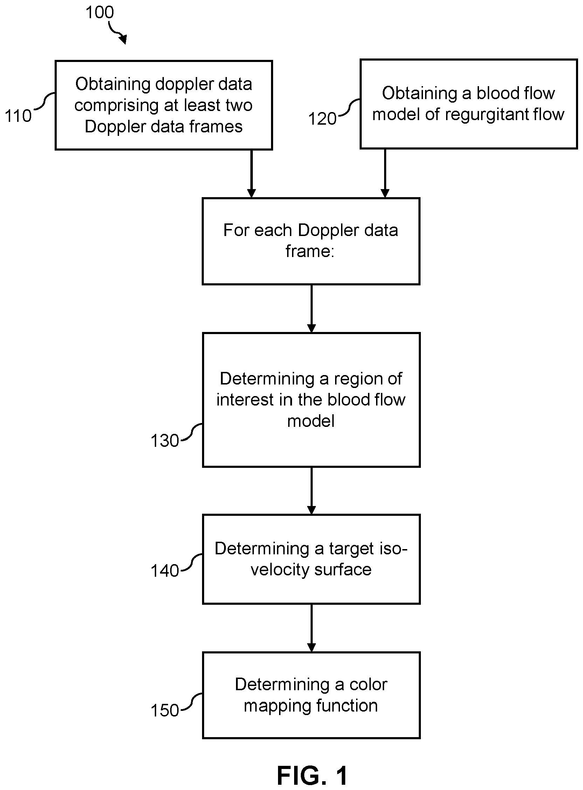

Referring now to Fig. 1, there is depicted a simplified flow diagram of a method 100 for dynamically determining a color mapping function for Doppler data of regurgitant flow in a cardiac valve according to a proposed embodiment.

The method 100 begins with the step 110 of obtaining Doppler data, wherein the Doppler data comprises at least two Doppler data frames from a temporal sequence and is representative of regurgitant flow associated with an orifice in a cardiac valve. Each Doppler data frame comprises velocity values. For example, the Doppler data can comprise three-dimensional (3D) color Doppler volume data of a cardiac valve throughout at least part of a cardiac cycle. A Doppler data frame can be understood as a single frame of Doppler data, i.e., describing a single moment in time, comprising velocity values.

In this embodiment, the at least two Doppler data frames from the temporal sequence are temporally adjacent. This allows for two adjacent frames from Doppler data, for example, to each have their own determined color mapping function such that, for example, subsequently generated color renderings (such as color maps) for the Doppler data are essentially being dynamically adjusted on a frame-by-frame basis. This essentially facilitates, for example, each frame of the Doppler data/video having an optimized color rendering. This is not essential for the invention, however, and in other embodiments, the Doppler data frames need not be temporally adjacent. For example, the method may comprise determining a color mapping function for every other Doppler data frame in a temporal sequence. In embodiments in which the Doppler data comprises a plurality of Doppler data frames, each Doppler data frame can be temporally adjacent.

Of course, as will be known to those skilled in the art of Doppler ultrasound, velocities measured using Doppler effect are only signed projections along the direction of the ultrasound beam.

Step 120 comprises obtaining a blood flow model of the regurgitant flow, wherein the blood flow model comprises a model of the orifice and blood flow velocity values through and around the orifice. For example, the blood flow model can comprise a 3D vector model of the orifice and blood flow through and around the orifice.

The following steps 130, 140, and 150 are then performed (independently) for each Doppler data frame of the Doppler data.

Step 130 comprises determining a region of interest in the blood flow model based on velocity values of the blood flow model and the Doppler data frame. In this embodiment, determining the region of interest is further based on a requirement that the region of interest is upstream of the cardiac valve. This facilitates adjusting the Doppler data frames based on regurgitant flow upstream of the cardiac valve, as opposed to flow downstream of the cardiac valve. This requirement, however, is not essential to the working of the invention.

Further, in this embodiment, determining the region of interest is further based on a requirement that the region of interest is within a predetermined distance of the cardiac valve. For example, the predetermined distance can be based on the limits of the blood flow model. This can ensure that the region of interest is close to the cardiac valve, thus providing more beneficial data for adjusting the Doppler data frames. Again, however, this requirement is not essential to the working of the invention.

In some embodiments, in subsequently generated color renderings/maps (using the determined color mapping functions), color noise can be removed in the region of interest as well as values likely to be aliased.

Step 140 comprises determining a target iso-velocity surface of the blood flow model based on data of the blood flow model within the region of interest. The target iso-velocity surface can also be understood as an optimal iso-velocity surface, wherein the optimal iso-velocity surface describes a proximal flow convergence zone of the regurgitant flow. In other words, the target iso-velocity surface can be understood as a surface which provides the best agreement between the velocity values of the Doppler data frame and the velocity values of the blood flow model, i.e., is guided by a best-fit between the Doppler data frame and the blood flow model.

For example, the blood flow model covers a spatial volumetric region of interest (VOI). The target iso-velocity surface (or shell) is just one potential subset of the VOI. There could therefore be a multiplicity of such iso-velocity surfaces/shells (each with a different velocity, for example) within the same VOI. A color map, for example, could then subsequently be designed in a way that multiple velocities could be represented simultaneously (e.g., stacking several red to blue scales).

In this embodiment, determining the target iso-velocity surface comprises determining the target iso-velocity surface of the blood flow model based on velocity values of the Doppler data within the region of interest. This facilitates the use of beneficial information for determining the target iso-velocity surface, but is not essential to the working of the invention.

Step 150 describes determining a color mapping function for the Doppler data frame based on the target iso-velocity surface, wherein the color mapping function is for mapping velocity values of the Doppler data frame to colors. For example, the color mapping function can comprise a set of colors, with each color being mapped to a velocity value from a set of velocity values. For instance, the color mapping function can indicate that a velocity of 5cm/s should be mapped to a blue color and a velocity of -5cm/s should be mapped to a red color, for example, with a gradient of colors between those two values. For example, a color mapping function for velocities can be designed to transition smoothly from cool hues (such as blue or green) for low speeds, gradually shifting towards warmer tones (like red or orange) as velocities increase, creating an intuitive visual representation where color correlates with the magnitude of speed. This approach allows users to quickly interpret velocity variations through a perceptually meaningful color spectrum.

In this embodiment, the color mapping function comprises a look-up table. This is an efficient and/or effective form of color mapping function. For instance, the look-up table can be determined such that an aliasing baseline of a color rendering/map generated using the look-up table can essentially be positioned at a beneficial position in the color rendering/map. For example, this can facilitate a transition of flow speed/direction to be made visible (or more visible) on the color map/rendering. In some embodiments, the look-up table can be determined such that a color rendering/map generated using the look-up table can comprise at least two aliasing baselines - this can provide a more volumetric impression of the velocity values. Of course, in other embodiments, the color mapping function can take any suitable form.

Further, in this embodiment, the color mapping function is configured to map at least one velocity value to a transparent color, i.e., to be transparent. This facilitates the generation of color renderings which can highlight, for example, a subset of velocity values or even a single velocity value by, for example, making other velocity values appear transparent in the color rendering, i.e., have no color. This is not essential to the invention, however, and in other embodiments, the color mapping function can not map any velocity values to be transparent, i.e., have no color.

Also, in this embodiment, determining the color mapping function comprises determining the color mapping function based on the velocity of the target iso-velocity surface. For example, the color mapping function can be determined such that, for example, an aliasing baseline of a subsequent color rendering of said Doppler data frame generated using said color mapping function is at the velocity of the target iso-velocity surface. This is not essential for the invention to work, however, and in other embodiments, determining the color mapping function can be based on other parameters of the target iso-velocity surface.

In this embodiment, the velocity of the target iso-velocity surface describes the average velocity of the region of interest. This is a beneficial parameter of the region of interest to use. In other embodiments, however, the velocity of the target iso-velocity surface can comprise the mean velocity of the region of interest, the mode velocity of the region of interest, the highest velocity of the region of interest, and/or the lowest velocity of the region of interest, etc.

This invention can then essentially facilitate a method for automatically adjusting, on a per-frame basis, aliasing baselines of a series of generated color maps/renderings (via the determined color mapping functions) for color flow data comprising a regurgitant flow from an orifice on a cardiac valve based on a model of the orifice geometry and PISA-like (Proximal Iso-velocity Surface Area) proximal flow convergence. In some embodiments, the PISA-like proximal flow convergence can be adapted to better match the color flow data before proceeding with the above-outlined method.

In accordance with the present invention, the method can thus comprise: receiving color flow data comprising a regurgitant flow from an orifice on a cardiac valve; receiving a model of said orifice geometry and PISA-like proximal flow convergence (adapted to the color flow data); and for each Doppler data frame of the color flow data: determining a region of interest in a vicinity of the orifice of the valve based on a comparison between the values of the color flow data and corresponding estimates obtained from the (adapted) model; determining a target iso-velocity surface of the (adapted) model based on the velocities for the voxels of the color flow data in the region of interest; and determining a color mapping function for the Doppler data frame based on the velocity of the target iso-velocity surface.

By automatically choosing a target (e.g., an optimal) iso-velocity value and determining a color mapping function per-frame, the invention approximates the information that the color flow data would show if an expert user had manually performed the PISA technique. In a preferred embodiment, the method can further comprise displaying the color flow data with a color rendering/map (generated using the determined color mapping function) side-by-side with a mesh representation of the proximal flow convergence of the (adapted) model. In this way, the invention can give a user more context and information regarding the automated measurement.

For instance, it should be clarified that it is not necessary that different velocities are mapped to different colors by the color mapping function nor is any monotony required. The color mapping function could alternatively be understood as a color mapping relation. For instance, if a plurality of target velocities and associated target iso-velocity surfaces are determined for the same volume of interest, then perhaps it might be desired to map all these different velocities to the same color intentionally. The color mapping function/relation could then facilitate this.

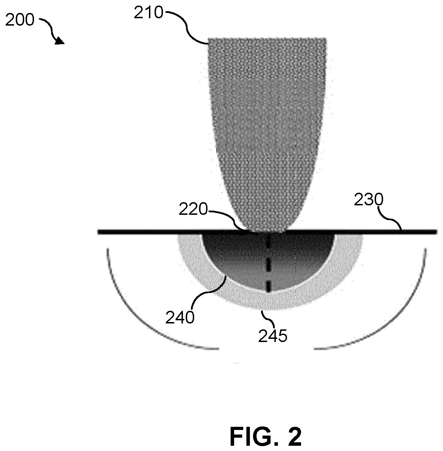

Referring now to Fig. 2, there is depicted a diagram 200 showing regurgitant flow in a cardiac valve 230. For example, an orifice 220 is provided in a cardiac valve 230 (such as a mitral valve). A liquid, such as blood or any other liquid, may thus pass from one side of the cardiac valve 230 to the other side through the orifice 220. Iso-velocity curves (i.e., potential target iso-velocity surfaces), 240 and 245, are positioned around the orifice 220. For example, positioned around the orifice 220 is a first iso-velocity curve 240 and a second iso-velocity curve 245. The iso-velocity curves, 240 and 245, can thus represent the velocity of the fluid as it approaches the orifice 220 before passing through it, resulting in a fluid jet 210.

For example, as the fluid approaches the orifice 220, it may accelerate, reaching a maximum velocity as it passes through the orifice 220. As a result, the fluid positioned at the first iso-velocity curve 240 may be of a constant velocity at all points along the curve 240. In addition, although the curves, 240 and 245, are shown as two-dimensional, the iso-velocity curves can be represented as three-dimensional hemispheric shaped shells representing all points within the fluid surrounding the orifice 220 of the same velocity.

The iso-velocity curves, 240 and 245, are observed upstream of the orifice 220. Fluid in this particular example may thus flow from below the cardiac valve 230 to above the cardiac valve 230.

For example, in clinical practice today, regurgitant flow quantification with ultrasound typically relies on the 2D Proximal Iso-velocity Surface Area (PISA) principle. The 2D PISA method employs a proximal flow convergence zone to measure the volume of regurgitation.

The principle underlying this method is straightforward and based on simple fluid dynamics. The flow convergence zone corresponds to regurgitant flow. Blood flow velocity increases as it approaches the regurgitant orifice 220. In the proximal flow convergence region, the blood flows through successive iso-velocity surfaces, 245 and 240, wherein iso-velocity means that the velocity on the surface of each individual iso-velocity surface is equal.

The inventors have realized that visual elements of the PISA method can be used to provide information to a user that otherwise would not have been available to them. The PISA method employs the proximal flow convergence zone to measure the volume of regurgitation. It assumes that the flow convergence zone corresponds to regurgitant flow. Blood flow velocity increases as it approaches the regurgitant orifice 220. As described above, the proximal flow convergence zone can thus be described as hemispheric shells, 240 and 245, in which the velocity on the surface of each of the shells is equal.

In the PISA method, which is usually performed in a single frame, the aliasing effect in color Doppler (e.g. Doppler color maps) is used. Because Pulse Wave Doppler is cyclic, there is an abrupt transition from yellow to blue, for example, that can easily be spotted in a frame (color map). In the PISA method, this artefact (the aliasing baseline) is used as a feature. By allowing the user to shift the baseline, such that the aliasing region is at a certain position, the user can determine the velocity for that position.

The inventors thus propose to significantly modify the PISA method in a number of ways. First, instead of treating the idea of a hemispheric iso-velocity shell as a mere concept, the algorithm returns an object representing the convergence zone of the algorithmic (blood flow) model. For example, it can be a triangular mesh resembling a hemispheric sphere (optimal iso-velocity shell). When intersecting this mesh with the view planes, a user can be provided with an overview over the convergence zone which the algorithm (blood flow model) based its results on. This is performed frame by frame.

Second, in the PISA method, it is the user which shifts the aliasing baseline. Here, instead, for each frame which the user reviews, a color mapping function can be automatically determined, essentially facilitating a shifting of the baseline to the velocity corresponding to the optimal iso-velocity shell for each frame by placing the aliasing artefact there. This enables the user to be provided with the region in the color Doppler data with this velocity (which can/will be different frame by frame). Essentially, this invention provides a dynamic (per-frame) automatic adaptation of the velocity transition in Doppler data frames of Doppler data.

A user can then be provided with the convergence zone as seen by the algorithm (blood flow model) side by side with a PISA-like aliasing transition for the same velocity. A user is then able to compare both and thus be provided with more context and information for assessing both results.

Therefore, while an algorithm (blood flow model) used need not share the many limitations of the PISA method and its often crude simplifications, the idea is to use clinicians' familiarity with the PISA method. The output of the model-based flow detection is done in such a way that it can be expressed in an optical language similar to PISA, but which contains important modifications such that it is not reducible to it. Using the model-based representation, optimal iso-velocity values can be extracted from the model for building the display of the regurgitant flow.

For instance, model-based algorithms can track and quantify cardiac regurgitation flow while building a regurgitant flow shape upstream the valve. This relies on assuming an orifice shape and distribution, and the corresponding upstream flow distribution. Comparing 3D color Doppler voxels with assumed flow models, the orifice extension can thus be iteratively updated and the resulting updated flow measured. The flow model can then be superimposed in 2D or 3D using meshes defined by specific iso-velocity thresholds.

In the present invention, the blood flow model can be formed by adding the contributions of a plurality of orifice elements. This model can be a 3D vector model. Each elemental contribution can be a vector field oriented towards this element with an amplitude modulated by (1/R²). This accumulated vector field can then be projected following the ultrasound beam orientation which simulates the color Doppler effect. The velocity amplitude of this `virtual Doppler' can then be adjusted in order to better correspond to the actual Doppler data.

In the present invention, the last described step can be used to define a target iso-velocity value which can then be applied to determine a color mapping function, for example, one which shows the aliasing velocity applied to a subsequently generated color map, and which can also be used to define the flow model shell. A region of interest in the flow model can be defined in order to compare the `virtual Doppler' with the actual Doppler data. This can be performed while adjusting the flow model to better match the Doppler data. This region of interest can be located upstream the valve and kept close to the valve. In this region, color noise can be removed as well as values likely to be aliased.

This region of interest (comprising only reliable voxels) can then be used to define a target iso-velocity value (and thus a surface). The optimal value for this velocity shell can be the average velocity from the region of interest or a combination of this value with the orifice velocity value. The most important fact is that this setting is done frame by frame (Doppler data frame by Doppler data frame), allowing a continuous adequate visualization of the regurgitant flow structure.

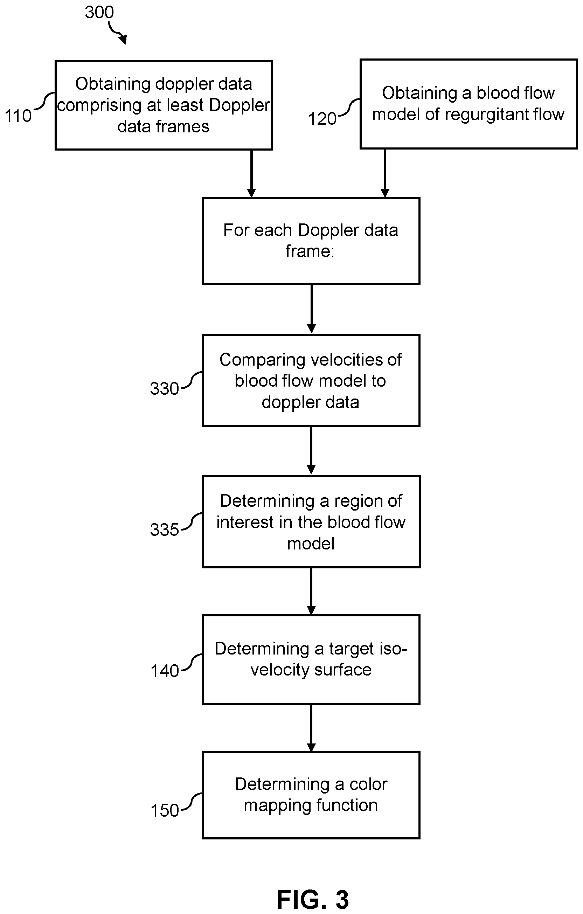

Referring now to Fig. 3, there is depicted a flow diagram of a method 300 for dynamically determining a color mapping function for Doppler data of regurgitant flow in a cardiac valve according to a proposed embodiment. Steps 110, 120, 140, and 150 are substantially the same as have been described in relation to method 100 in Fig. 1.

Steps 330, 335, 140, and 150 are performed (independently) for each Doppler data frame of the Doppler data. Step 330 comprises comparing velocity values of the blood flow model to velocity values of the Doppler data frame, and step 335 comprises determining a region of interest in the blood flow model based on the comparison. In this way, for example, the region where the different velocities best match, i.e., the region where the voxels of the blood flow model are most reliable, can be used to determine a target iso-velocity surface.

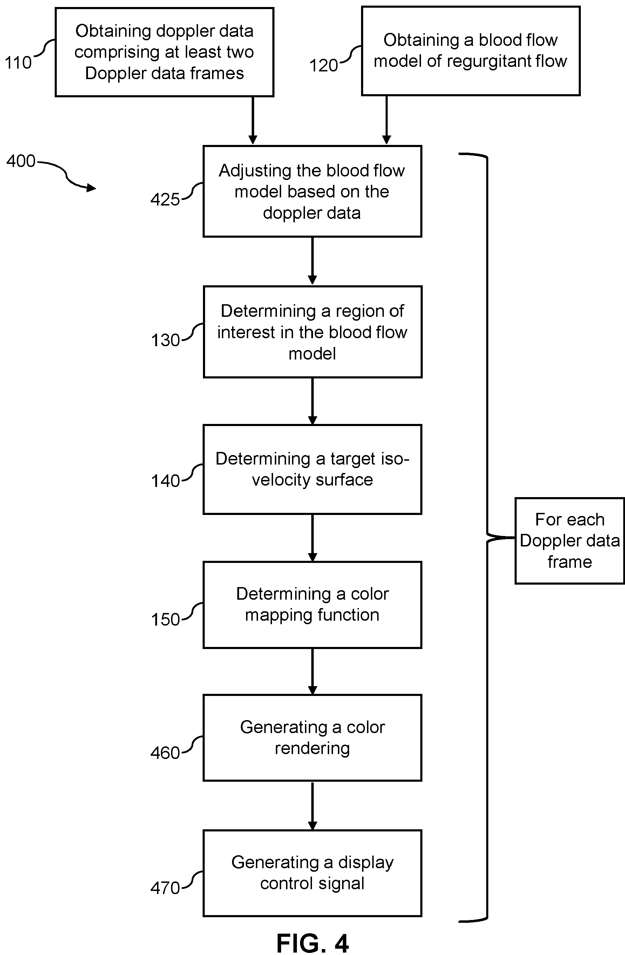

Referring now to Fig. 4, there is depicted a flow diagram of a method 300 for dynamically determining a color mapping function for Doppler data of regurgitant flow in a cardiac valve according to a proposed embodiment. Steps 110, 120, 130, 140, and 150 are substantially the same as have been described in relation to method 100 in Fig. 1.

Steps 425, 130, 140, 150, 460, and 470 are performed (independently) for each Doppler data frame of the Doppler data. Step 425 comprises adjusting the blood flow model based on the Doppler data. This allows the blood flow model to be adjusted to, for example, better correspond to the actual Doppler data. In other words, the blood flow model can be compared to actual Doppler data (frames), and then adjusted such that the blood flow model better matches the actual flow seen in the Doppler data to ensure accuracy. For instance, the blood flow model can be adjusted for each Doppler data frame to better match each said Doppler data frame.

Step 460 comprises generating a color rendering for a Doppler data frame based on the velocity values of said Doppler data frame and the determined color mapping function for said Doppler data frame. In this way, a user can be provided with a color rendering generated using a more beneficial/optimal color mapping function.

Step 470 comprises generating a display control signal, the display control signal describing the color rendering for the Doppler data frame. In this way, a user can be presented with a generated color rendering and, optionally, the color mapping function of said Doppler data frame. In this way, a user can analyze the color rendering in the context of the color mapping function which might be, for instance, a look-up table for a color map, to see, for example, what velocity the aliasing baseline is representing. Further, in this embodiment, the display control signal further describes the Doppler data. In this way, a user can be presented with the actual Doppler data as well as the generated color rendering/map for better context and understanding of both.

In some embodiments, the method 400 can further comprise generating a mesh representation of a proximal flow convergence of the blood flow model. The display control signal of step 470 can thus further describe the mesh representation. In this way, a user can be presented with further useful representations of the data for better context and understanding.

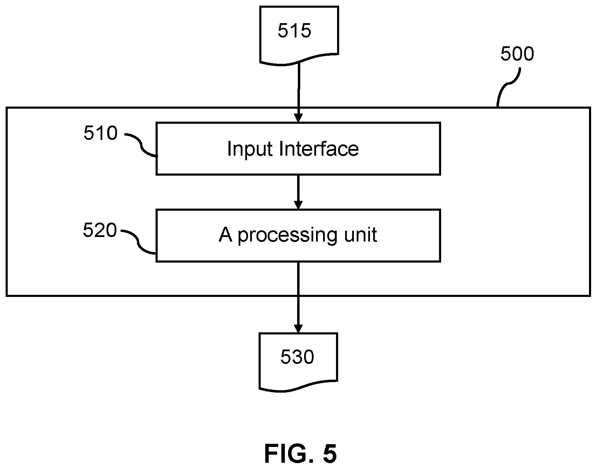

Referring now to Fig. 5, there is depicted a system 500 for dynamically determining a color mapping function for Doppler data of regurgitant flow in a cardiac valve according to a proposed embodiment. The system 500 comprises an input interface 510 and a processing unit 520.

The system 500 is configured to adjust Doppler data frames of regurgitant flow in a cardiac valve by processing inputs 515. The inputs 515 comprise Doppler data, wherein the Doppler data comprises at least two Doppler data frames from a temporal sequence and is representative of regurgitant flow associated with an orifice in a cardiac valve, and wherein each Doppler data frame comprises velocity values; and a blood flow model of the regurgitant flow, wherein the blood flow model comprises a model of the orifice and blood flow velocity values through and around the orifice. The input interface 510 obtains the inputs 515 and the processing unit 520 is then configured to process the inputs 515 to generate an output 530. The processing unit 520 is configured to, for each Doppler data frame of the Doppler data, determine a region of interest in the blood flow model based on velocity values of the blood flow model and the Doppler data frame; determine a target iso-velocity surface of the blood flow model based on data of the blood flow model within the region of interest; and determine a target iso-velocity surface of the blood flow model based on data of the blood flow model within the region of interest; and determining a color mapping function for the Doppler data frame based on the target iso-velocity surface, wherein the color mapping function is for mapping velocity values of the Doppler data frame to colors. The output 530 thus comprises a color mapping function for each Doppler data frame of the Doppler data.

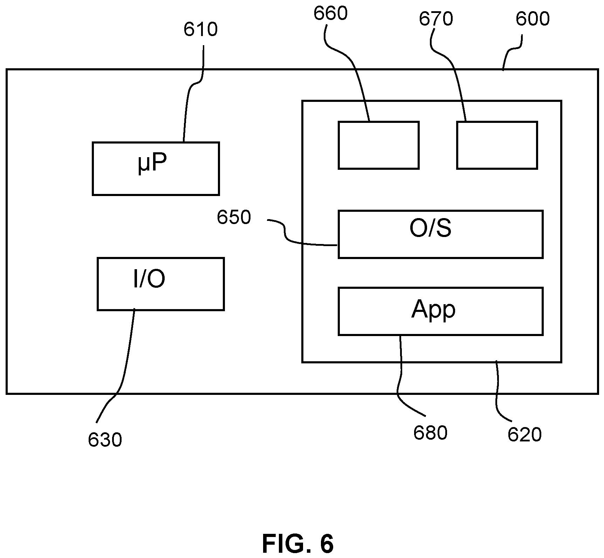

Fig. 6 illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620 and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at start-up, start the O/S 650, and support the transfer of data among the hardware devices. The BIOS is stored in some type of read-only-memory, such as ROM, PROM, EPROM, EEPROM or the like, so that the BIOS can be executed when the computer 800 is activated.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1 and 3-4, and the system of Fig. 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for dynamically determining a color mapping function for Doppler data of regurgitant flow in a cardiac valve, the method comprising:
obtaining Doppler data (110), wherein the Doppler data comprises at least two Doppler data frames from a temporal sequence and is representative of regurgitant flow associated with an orifice in a cardiac valve, and wherein each Doppler data frame comprises velocity values;
obtaining a blood flow model of the regurgitant flow (120), wherein the blood flow model comprises a model of the orifice and blood flow velocity values through and around the orifice; and
for each Doppler data frame of the Doppler data:
determining a region of interest in the blood flow model (130) based on velocity values of the blood flow model and the Doppler data frame;
determining a target iso-velocity surface of the blood flow model (140) based on data of the blood flow model within the region of interest; and
determining a color mapping function for the Doppler data frame (150) based on the target iso-velocity surface, wherein the color mapping function is for mapping velocity values of the Doppler data frame to colors.

2. The computer-implemented method of claim 1, wherein the at least two Doppler data frames from the temporal sequence are temporally adjacent.

3. The computer-implemented method of any of claims 1 or 2, wherein the method further comprises: generating a color rendering (460) for a Doppler data frame based on the velocity values of said Doppler data frame and the determined color mapping function for said Doppler data frame.

4. The computer-implemented method of claim 3, wherein the method further comprises: generating a display control signal (470), the display control signal describing the color rendering for the Doppler data frame and, optionally, the color mapping function of said Doppler data frame.

5. The computer-implemented method of any prior claim, wherein the color mapping function comprises a look-up table.

6. The computer-implemented method of any prior claim, wherein the color mapping function is configured to map at least one velocity value to a transparent color.

7. The computer-implemented method of any prior claim, wherein determining a region of interest in the blood flow model comprises:
comparing velocity values of the blood flow model to velocity values of the Doppler data frame (330); and
determining a region of interest in the blood flow model (335) based on the comparison.

8. The computer-implemented method of any prior claim, wherein determining the color mapping function of the Doppler data frame comprises determining the color mapping function based on the velocity of the target iso-velocity surface.

9. The computer-implemented method of claim 8, wherein the velocity of the target iso-velocity surface describes the average velocity of the region of interest.

10. The computer-implemented method of any prior claim, wherein the region of interest is further based on a requirement that the region of interest is upstream of the cardiac valve.

11. The computer-implemented method of any prior claim, wherein the region of interest is further based on a requirement that the region of interest is within a predetermined distance of the cardiac valve.

12. The computer-implemented method of any prior claim, wherein, prior to determining a region of interest, the method further comprises adjusting the blood flow model (425) based on the Doppler data.

13. The computer-implemented method of any prior claim, wherein determining a target iso-velocity surface comprises determining a target iso-velocity surface of the blood flow model based on velocity values of the Doppler data within the region of interest.

14. A computer program comprising code means for implementing the method of any preceding claim when said program is run on a processing system.

15. A system (500) for dynamically determining a color mapping function for Doppler data of regurgitant flow in a cardiac valve, the system comprising:
an input interface (510) configured to:
obtain Doppler data, wherein the Doppler data comprises at least two Doppler data frames from a temporal sequence and is representative of regurgitant flow associated with an orifice in a cardiac valve, and wherein a Doppler data frame comprises velocity values;
obtain a blood flow model of the regurgitant flow, wherein the blood flow model comprises a model of the orifice and blood flow velocity values through and around the orifice; and
a processing unit (520) configured to:
for each Doppler data frame of the Doppler data:
determine a region of interest in the blood flow model based on velocity values of the blood flow model and the Doppler data frame;
determine a target iso-velocity surface of the blood flow model based on data of the blood flow model within the region of interest; and
determining a color mapping function for the Doppler data frame based on the target iso-velocity surface, wherein the color mapping function is for mapping velocity values of the Doppler data frame to colors.
